# EUROPÄISCHE PATENTSCHRIFT

(11) **EP 0 919 555 B1**
(45) Veröffentlichungstag und Bekanntmachung des Hinweises auf die Patenterteilung: **08.05.2002**
(21) Anmeldenummer: 98122131.0
(22) Anmeldetag: 24.11.1998
(51) Int. Cl.: C07D 487/04, C07D 239/06

(54) **Verfahren zur Herstellung von bicyclischen Amidinen, Diazacycloalkene und ein Verfahren zu deren Herstellung**
Process for the preparation of bicyclic amidines, diazacycloalkenes and a process for their preparation
Procédé pour la préparation d'amidines bicycliques, diazacycloalcènes et un procédé pour leur préparation

(30) Priorität: 28.11.1997 DE 19752935
(43) Veröffentlichungstag der Anmeldung: 02.06.1999
(73) Patentinhaber: BASF AKTIENGESELLSCHAFT, 67056 Ludwigshafen (DE)
(72) Erfinder: Friedrich, Wolfgang, 67346 Speyer (DE); Kneuper, Heinz-Josef, Dr., 68163 Mannheim (DE); Eller, Karsten, Dr., 67061 Ludwigshafen (DE); Witzel, Tom, Dr., 67069 Ludwigshafen (DE)
(74) Vertreter: Isenbruck, Günter, Dr.

(56) Entgegenhaltungen:
- EP-A- 0 382 056
- EP-A- 0 662 476
- DE-A- 1 545 855
- DE-C- 730 182

## Beschreibung

Die vorliegende Erfindung betrifft ein Verfahren zur Herstellung eines bicyclischen Amidins, in dessen Verlauf als Zwischenstufe ein Diazacycloalken entsteht, sowie ein Diazacycloalken, ein Verfahren zu dessen Herstellung und dessen Verwendung zur Herstellung eines bicyclischen Amidins.

Bicyclische Amidine, insbesondere Diazabicycloalkene, werden in organisch chemischen Reaktionen häufig als starke Basen eingesetzt. So wurden diese Verbindungen ursprünglich durch ihre Fähigkeit bekannt, Dehydrohalogenierungsreaktionen zu unterstützen, was zuerst bei der Vitamin A Synthese angewandt wurde. Die bicyclischen Amidine lassen sich jedoch auch bei anderen Reaktionen einsetzen, wobei häufig ein im Vergleich zum Einsatz konventioneller Basen milderer Reaktionsverlauf, eine höhere Selektivität und häufig auch eine höhere Ausbeute resultiert.

Bicyclische Amidine können nach verschiedenen Methoden hergestellt werden. Eine bekannte Synthesemethode verläuft zunächst über die Addition von Acrylnitril an ein Lactam, wobei sich ein Cyanoethyl-Lactam bildet. Dieses Lactam wird anschließend zu Aminopropyl-Lactam hydriert und in einem letzten Schritt unter saurer Katalyse und Wasserabspaltung zum bicyclischen Amidin cyclisiert (Oedinger et al., Synthesis (1972) S. 591- 598; idem, Chem. Ber. 99 (1966) S. 2012- 2016 und Xing - Quan, J. Nat. Gas Chem. 4 (1995) S. 119 - 127). Der Nachteil dieses Verfahrens liegt in seiner Mehrstufigkeit, sowie in der Notwendigkeit, als Reagens das stark toxische Acrylnitril zu verwenden.

Der Einsatz von Acrylnitril kann jedoch durch eine zweistufige Herstellung des bicyclischen Amidins ausgehend von Lactonen vermieden werden. Die DE-PS 730 182 beschreibt die Herstellung von Aminoalkylpyrrolidonen ausgehend von Lactonen und Diaminen, durch mehrstündiges Erhitzen im Autoklaven. Das so erhaltene Produkt kann dann analog dem oben beschriebenen Verfahren sauer katalysiert (z. B. nach DE-PS 1 545 855) zu bicyclischen Amidinen cyclisiert werden.

Die EP-A 0 662 476 kombiniert diese beiden Schritte und offenbart ein Verfahren zur Herstellung bicyclischer Amidine durch Erhitzen von Lactonen und Diaminen unter Zusatz eines sauren Katalysators im Autoklaven. Nachteilig wirkt sich dabei die Notwendigkeit aus, unter erhöhtem Druck zu arbeiten. Es werden teure Druckapparaturen benötigt, die zudem aufgrund der beim offenbarten Verfahren notwendigen sauren Katalyse aus speziellen säurebeständigen Materialien angefertigt sein müssen. Ein analoges Verfahren beschreiben Nakatani et al. in Chem. Expr. 8 (1993) S. 825 - 828.

Die DE-A 35 12 627 beschreibt die Umsetzung von Lactonen wie Butyrolacton mit Aminen wie 1,3-Diaminopropan und Säuren wie Oxalsäure zu offenkettigen Amidsalzen wie 3-(4-Hydroxybutanoylamino)-propyl-ammonium-oxalat. Lin und Gromelski (Proc. SPI Annu. Tech./Mark. Conf (1984), S 138 - 141) beschreiben ebenfalls die Herstellung offenkettiger Amide vom Typ N,N'-(1,ω-Alkylen)-bis-4-hydroxybutyramid bei der Umsetzung von Butyrolacton mit Diaminen, die bei höherer Temperatur zu Alkylen-bis-pyrrolidonen cyclisieren. Lagerman et al. (J. Am. Oil Chem. Soc. **71** (1994), S. 97 - 100) beschreiben die Bildung offenkettiger Amide bei der Reaktion von Diaminen mit primärer und sekundärer Aminogruppe mit Butyro- oder Caprolacton.

Die EP-A 0 382 056 beschreibt die Umsetzung von Lactamen mit Diaminen in der Gasphase an Zeolithen bei erhöhten Temperaturen und Normaldruck. Die erreichbaren Ausbeuten an bicyclischen Amidinen sind jedoch unbefriedigend und die Druckschrift gibt keine Information über die Standzeit der Zeolithkatalysatoren.

Der vorliegenden Erfindung lag damit die Aufgabe zugrunde, ein Verfahren zur Herstellung eines bicyclischen Amidins zur Verfügung zu stellen, das ohne den aufwendigen Einsatz von Druckapparaturen auskommt, bei dem eine Verwendung saurer Katalysatoren nicht notwendig ist, und das in hohen Ausbeuten zum gewünschten bicyclischen Amidin führt.

Die Aufgabe wurde dadurch gelöst, daß ein Lacton und ein Diamin unter Entfernung des Reaktionswassers während des Reaktionsverlaufs zum bicyclischen Amidin umgesetzt wurden.

Gegenstand der Erfindung ist damit ein Verfahren zur Herstellung eines bicyclischen Amidins der allgemeinen Formel I 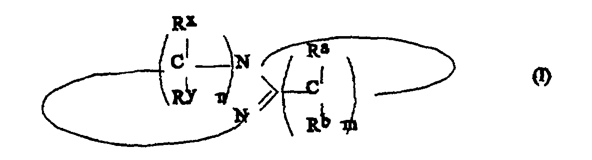 worin R^{x}, R^{y}, R^{a} und R^{b} jeweils unabhängig voneinander für Wasserstoff, C₁₋₁₀-Alkyl, C₂₋₁₀-Alkenyl, C₂₋₁₀-Alkinyl oder C₆₋₁₂-Aryl, wobei die Reste ihrerseits noch mit Hydroxy-, Amino-, C₁₋₄-Alkylamino- oder Mercaptogruppen substituiert sein können, stehen und n und m unabhängig voneinander fiir Zahlen von 2 bis 12 stehen, wobei a, b, x und y jeweils die Reste R am jeweiligen Kohlenstoffatom 1 bis m bzw. 1 bis n indizieren,
bei dem ein Reaktionsgemisch, enthaltend ein Lacton der allgemeinen Formel II 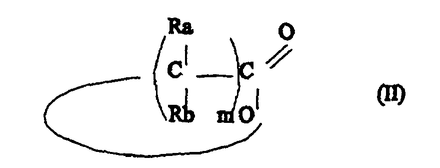 worin R^{a}, R^{b} und m jeweils die oben genannte Bedeutung haben,
und ein Diamin der allgemeinen Formel III 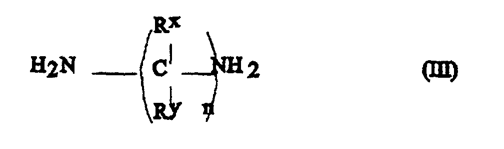 worin R^{x}, R^{y} und n jeweils die oben genannte Bedeutung haben,
unter Wasserabspaltung zur Reaktion gebracht wird, wobei durch die Wasserabspaltung entstandenes Wasser während der Reaktion aus dem Reaktionsgemisch entfernt wird.

Mit Hilfe des erfindungsgemäßen Verfahrens können bicyclische Amidine der allgemeinen Formel I hergestellt werden, die vielfältige Einsatzmöglichkeiten bei der organisch chemischen Synthese, insbesondere bei der Dehydrohalogenierung organischer Verbindungen oder bei Polymersynthesen erfüllen können.

Das im Rahmen des erfindungsgemäßen Verfahrens herstellbare bicyclische Amidin der allgemeinen Formel I weist Reste R^{x}, R^{y}, R^{a} und R^{b} auf, die unabhängig voneinander für Wasserstoff, C₁₋₁₀-Alkyl, C₂₋₁₀-Alkenyl oder C₂₋₁₀-Alkinyl stehen können. Die Reste R^{x}, R^{y}, R^{a} und R^{b} können jeweils unabhängig voneinander linear oder gegebenenfalls verzweigt sein oder können jeweils mindestens paarweise gemeinsam ein Ringsystem bilden.

Das bicyclische Amidin der allgemeinen Formel I weist zwei Ringsegmente auf, von denen das erste zwei Stickstoffatome und die mit der Variablen n bezifferten Gruppen CR^{x}R^{y} und das zweite nur ein Stickstoffatom (gemeinsam mit dem ersten Ringsegment) und die mit der Variablen m bezifferten Gruppen CR^{a}R^{b} enthält.

Das Lacton der allgemeinen Formel II weist einen Ring auf, der die mit der Variablen m bezifferten Gruppen CR^{a}R^{b} enthält, die den Gruppen CR^{a}R^{b} des zweiten Ringsegments im bicyclischen Amidin entsprechen.

Die Variablen n und m stehen damit für die Anzahl der durchgängig durch C-C-Bindungen miteinander verbundenen Gruppen CR^{a}R^{b} bzw. CR^{x}R^{y} im jeweiligen Ring bzw. Ringsegment der allgemeinen Formeln I und II, bzw. für die Zahl der durchgängig durch C-C-Bindungen verbundenen Kohlenstoffatome CR^{x}R^{y} zwischen den Aminogruppen des Diamins der allgemeinen Formel III.

Die Variablen n und m bezeichnen damit im Falle des bicyclischen Amidins die jeweiligen, im Ringsegment durch die genannten Variablen gekennzeichneten und durchgehend verbundenen Kohlenstoffatome und indizieren jedes der durch n oder m gekennzeichneten Kohlenstoffatome im bicyclischen Amidin der allgemeinen Formel I mit einer Zahl von 1 bis n (für die mit n gekennzeichneten Kohlenstoffatome) bzw. n+1 bis n+m (fiir die mit m gekennzeichneten Kohlenstoffatome).

Im Falle des Lactons der allgemeinen Formel II bezeichnet die Variable m die im Ring entsprechend gekennzeichneten und durchgehend verbundenen Kohlenstoffatome und indiziert jedes der durch m gekennzeichneten Kohlenstoffatom mit einer Zahl von 1 bis m.

Im Falle des Diamins der allgemeinen Formel III bezeichnet die Variable n die entsprechend gekennzeichneten und durchgehend verbundenen Kohlenstoffatome zwischen den Aminogruppen und indiziert jedes der durch n gekennzeichneten Kohlenstoffatom mit einer Zahl von 1 bis n.

Die Variablen a, b, x und y indizieren jeweils die Reste R am jeweiligen Kohlenstoffatom 1 bis n bzw. 1 bis m bzw. n+1 1 bis n+m. Steht beispielsweise n fiir 4, so steht x für die Zahlen 1, 3, 5 und 7, jeweils entsprechend dem Rest R¹ am Kohlenstoffatom 1, R³ am Kohlenstoffatom 2, R⁵ am Kohlenstoffatom 3 und R⁷ am Kohlenstoff 4. Entsprechend steht y für die Zahlen 2, 4, 6 und 8, entsprechend dem Rest R² am Kohlenstoff 1, R⁴ am Kohlenstoffatom 2, R⁶ am Kohlenstoffatom 3 und R⁸ am Kohlenstoffatom 4. Für die Variablen a und b gilt die gleiche, vom Wert der Variablen m abhängige, Zuordnung.

Steht in der allgemeinen Formel II m beispielsweise für die Zahl 3, so entspricht die Formel (-CR^{a}R^{b})ₘ- dem Rest -CR¹R²-CR³R⁴-CR⁵R⁶-, steht m für die Zahl 5, so entspricht die Formel (-CR^{a}R^{b})ₘ- dem Rest -CR¹R²-CR³R⁴-CR⁵R⁶-CR⁷R⁸-CR⁹R¹⁰. Die Reste R¹ bis R¹⁰ entsprechen dabei der oben angegebenen Definition für R^{a} und R^{b}. Entsprechendes gilt auch fiir die Reste R^{x} und R^{y}. Steht beispielsweise n in der allgemeinen Formel III für die Zahl 3, so entspricht die Formel (-CR^{x}R^{y})ₙ-dem Rest -CR¹R²-CR³R⁴-CR⁵R⁶-.

Steht in einer Verbindung der allgemeinen Formel I oder IV beispielsweise n für die Zahl 3 und m für die Zahl 3, so entspricht die Formel (-CR^{x}R^{y})ₙ-dem Rest -CR¹R²-CR³R⁴-CR⁵R⁶- und die Formel (-CR^{a}R^{b})ₘ- dem Rest -CR⁷R⁸-CR⁹R¹⁰-CR¹¹R¹²-. Somit ist eine eindeutige Zuordnung der jeweiligen Reste gegeben.

Es ist im Rahmen der vorliegenden Erfindung bevorzugt, wenn n fiir Werte von 2 bis 6 und m für Werte von 3 bis 7 steht. Besonders bevorzugt ist es, wenn n für Werte von 2 bis 4 und m fiir Werte von 3 bis 5 steht, wobei in einer ganz besonders bevorzugten Asführungsform n fiir den Wert 3 steht.

Es ist im Rahmen der vorliegenden Erfindung ebenfalls bevorzugt, wenn die Reste R^{x}, R^{y}, R^{a} und R^{b} unabhängig voneinander für Wasserstoff, Hydroxygruppen, oder für C₁₋₄-Alkyl steht, wobei es besonders bevorzugt ist, wenn alle Reste R^{x}, R^{y}, R^{a} und R^{b} für Wasserstoff stehen.

Die Herstellung der bicyclischen Amidine der allgemeinen Formel I wird mittels eines Reaktionsgemischs durchgeführt, das ein Lacton der allgemeinen Formel II enthält.

Da das Lacton in einen Bestandteil des bicyclischen Amidins umgewandelt wird, entsprechen die im Lacton vorliegenden Reste R^{a} und R^{b} den jeweiligen im gewünschten bicyclischen Amidin vorliegenden Resten. Demnach ist es bevorzugt, wenn R^{a} und R^{b} unabhängig voneinander fiir Wasserstoff, Hydroxygruppen, Aminogruppen oder C₁₋₄-Alkyl, insbesondere für Wasserstoff, stehen. Die Variable m steht entsprechend bevorzugt fiir Zahlen von 3 - 8, insbesondere für die Zahl 3.

Als Lactone der allgemeinen Formel II werden im Rahmen der vorliegenden Erfindung vorzugsweise γ-Butyrolacton, γ- oder δ-Valerolacton, γ-, δ- oder ε-Caprolacton oder andere substituierte Lactone, beispielsweise Pantolacton (2-Hydroxy-3,3-dimethylbutyrolacton) eingesetzt.

Als weitere Komponente enthält das im erfindungsgemäßen Verfahren zur Herstellung bicyclischer Amidine eingesetzte Reaktionsgemisch ein Diamin der allgemeinen Formel III.

Da auch das Diamin Bestandteil des bicyclischen Amidins wird, entsprechen die Reste R^{x} und R^{y} den im gewünschten bicyclischen Amidin der allgemeinen Formel I vorliegenden Resten. Bevorzugt stehen R^{x} und R^{y} unabhängig voneinander für Wasserstoff oder C₁₋₄-Alkyl, insbesondere für Wasserstoff.

Die Variablen x und y indizieren jeweils die Reste R am jeweiligen Kohlenstoffatom 1 bis n. Die Indizierung erfolgt daher gemäß dem für die Variablen a und b oben angegebenen Beispiel.

Als Diamine der allgemeinen Formel III kommen beispielsweise 1,2-Diaminoethan, 1,2-Diaminopropan oder 1,3-Diaminopropan, 1,3-Diaminobutan, 1,4-Diaminobutan, 1,2-Diaminocyclohexan, 2,n-Diaminoalkane wie 2,3-Diaminobutan oder verzweigte Diamine, deren Aminogruppen durch 2 bis etwa 8, insbesondere 2 bis 4 C-Atome getrennt sind, in Frage. Besonders bevorzugt sind 1,2-Diaminoethan und 1,3-Diaminopropan.

Zu Beginn der Reaktion beträgt der Anfangswert für das molare Verhältnis von Lactonen zu Diaminen etwa 1 oder ist kleiner als 1, vorzugsweise beträgt das Anfangsverhältnis von Lacton zu Diamin mindestens 1:1,5. Eine Obergrenze für den Anteil an Diamin, d. h. eine Untergrenze für das Lacton : Diamin-Verhältnis muß nicht notwendigerweise eingehalten werden. Aus grundsätzlichen Überlegungen kann es jedoch sinnvoll sein, die Menge des eingesetzten Diamins im Verhältnis zum Lacton zu begrenzen, d. h. beispielsweise ein maximales Lacton : Diamin-Verhältnis von 1:20, 1:10, 1:8, 1:4, 1:3 oder 1:2 einzuhalten.

Das erfindungsgemäße Verfahren zeichnet sich dadurch aus, daß während der Reaktionsdauer durch die Wasserabspaltung entstandenes Wasser aus dem Reaktionsgemisch entfernt wird. Es ist dabei vorteilhaft, wenn die Entfernung des Wassers nicht nur an einem einzigen bestimmten Punkt des Reaktionsverlaufs erfolgt, sondern an zwei oder mehreren beispielsweise in gleichen Zeitabständen aufeinanderfolgenden Zeitpunkten während des Reaktionsverlaufs. Es ist ebenso möglich, daß die Entfernung des durch die Wasserabspaltung entstandenen Wassers kontinuierlich über einen Zeitraum erfolgt, der mindestens der Hälfte der gesamten Reaktionsdauer entspricht.

In einer besonders bevorzugten Ausführungsform des erfindungsgemäßen Verfahrens erfolgt die Entfernung des Wassers aus dem Reaktionsgemisch kontinuierlich während der gesamten Reaktionsdauer.

Zur Entfernung des Wassers aus dem Reaktionsgemisch bieten sich die üblichen im Rahmen der organisch chemischen Synthese angewandten Verfahren an. Insbesondere wird die Entfernung des Wassers aus dem Reaktionsgemisch durch Destillation erreicht, wobei es vorteilhaft ist, wenn im Reaktionsgemisch wenigstens eine Komponente vorliegt, die als Schleppmittel für das aus dem Reaktionsgemisch zu entfernende Wasser dient und damit das durch die Wasserabspaltung entstandene Wasser mit Hilfe eines Schleppmittels entfernt wird.

Als Schleppmittel sind alle diejenigen Verbindungen geeignet, deren Siedepunkt so hoch liegt, daß ein Einsatz des Schleppmittels bei den bei der Umsetzung herrschenden Temperaturen noch möglich ist und die gegenüber den Verbindungen I bis IV ein inertes Verhalten zeigen. Der Siedepunkt des Schleppmittels sollte mindestens 120°C, vorzugsweise jedoch darüber betragen. Als Schleppmittel geeignet sind insbesondere alle mit Wasser ein Azeotrop bildende Verbindungen, z.B. flüssige, polycyclische Kohlenwasserstoffe wie Tetralin oder Decalin.

Besonders bevorzugt ist es jedoch im Rahmen der vorliegenden Erfindung, wenn als Schleppmittel für das aus dem Reaktionsgemisch zu entfernende Wasser das zur Umsetzung eingesetzte Diamin verwendet wird. In der Regel lassen sich Diamine ausgezeichnet als Schleppmittel für Wasser nutzen. In einer besonders bevorzugten Ausführungsform der Erfindung wird daher das während der Reaktion durch Wasserabspaltung entstandene Wasser mit Hilfe des im Reaktionsgemisch vorliegenden Diamins der allgemeinen Formel III entfernt.

Da auf diese Weise dem Reaktionsgemisch ständig zur Umsetzung benötigtes Diamin entzogen wird, sollte dem Reaktionsgemisch in dem Maße wieder Diamin zugeführt werden, in dem es durch Destillation der Umsetzung entzogen wird. Dabei sollte mindestens soviel Diamin dem Reaktionsgemisch zugeführt werden, wie fiir eine vollständige Umsetzung des im Reaktionsgemisch vorliegenden, noch nicht umgesetzten Lactons der allgemeinen Formel II nötig ist. Mit anderen Worten, im Reaktionsgemisch sollte zu jedem Zeitpunkt mindestens ein äquimolares Verhältnis aus nicht umgesetztem Lacton der allgemeinen Formel II und Diamin der allgemeinen Formel III vorliegen.

Ein weiteres Kriterium für die Menge des während des Reaktionsverlaufs zugeführten Diamins der allgemeinen Formel III besteht darin, daß zu jedem Zeitpunkt der Reaktion eine ausreichende Entfernung des bei der Umsetzung durch Wasserabspaltung entstehenden Wassers möglich sein sollte. Da die Menge des entstehenden Wassers im Verlauf der Reaktion abnimmt, kann auch die Menge an zugeführtem Diamin während des Reaktionsverlaufs abnehmen.

Die Reaktionstemperatur bei der Umsetzung beträgt in Abhängigkeit von den im Reaktionsgemisch vorliegenden, miteinander umzusetzenden Komponenten, nämlich dem Lacton der allgemeinen Formel II und dem Diamin der allgemeinen Formel III, zwischen etwa 80°C und etwa 350°C, vorzugsweise etwa 130°C bis 350°C. Niedrige Reaktionstemperaturen von beispielsweise etwa 80°C, 100°C, 120°C bis zu etwa 150°C führen in der Regel dann zum gewünschten Produkt, wenn fiir eine ausreichend lange Reaktionszeit gesorgt wird. In der Regel ist es im Rahmen des erfindungsgemäßen Verfahrens vorteilhaft, wenn höhere Reaktionstemperaturen, beispielsweise 170°C, 190°C, 200°C, 210°C oder sogar darüber, eingehalten werden. Gute Ergebnisse lassen sich beispielsweise bei Lactonen und Diaminen, die beispielsweise aus sterischen Gründen oder wegen der abnehmenden Entropie des Ringschlusses nur schwer miteinander in Reaktion zu bringen sind, dann erzielen, wenn die Reaktionstemperatur beispielsweise etwa 220°C bis etwa 300°C, insbesondere etwa 240°C oder etwa 250°C beträgt. In einer bevorzugten Ausführungsform des erfindungsgemäßen Verfahrens liegt die Reaktionstemperatur bei etwa 120°C bis etwa 220°C.

Die Reaktionsdauer beträgt in Abhängigkeit von der Reaktionstemperatur mehr als etwa 3 Stunden, beispielsweise etwa 5 Stunden bis etwa 100 Stunden, vorzugsweise etwa 10 Stunden bis etwa 80 Stunden, und insbesondere etwa 24 Stunden bis etwa 60 Stunden.

In einer bevorzugten Ausführungsform der Erfindung wird die Umsetzung bei etwa 180 bis etwa 210°C und einer Reaktionsdauer von etwa 24 bis etwa 72, insbesondere etwa 48 Stunden durchgeführt.

Beispiele für erfindungsgemäß herstellbare, bicyclische Amidine sind 1,5-Diazabicyclo[4,3,0]nonen-5 (DBN), 1,8-Diazabicyclo[5.4.0]undecen-7 (DBU), 1,6-Diazabicyclo[5.5.0]dodecen-6, 1,7-Diazabicyclo[6.5.0]tridecen-7, 1,8-Diazabicyclo[7.4.0]tridecen-8, 1,8-Diazabicyclo[7.5.0]tetradecen-8, 1,5-Diazabicyclo[4.4.0]decen-5 (DBD), 1,8-Diazabicyclo[5.3.0]decen-7, 1,10-Diazabicycio[7.3.0]dodecen-9, 1,10-Diazabicyclo[7.4.0]tridecen-9 oder die entsprechend substituierten Derivate dieser Verbindungen. Besonders bevorzugt sind DBN, DBD oder DBU oder ein Gemisch aus zwei oder mehr davon.

Eine Besonderheit des erfindungsgemäßen Verfahrens liegt darin, daß es über ein (isolierbares) Zwischenprodukt in Form eines Diazacycloalkens der allgemeinen Formel IV 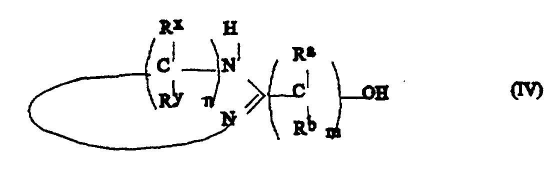 verläuft, wobei dieses Zwischenprodukt durch eine besondere, ebenfalls im Rahmen der vorliegenden Erfindung nachfolgend offenbarte Reaktionsführung isoliert werden kann. Es wird angenommen, daß die Bildung des Zwischenprodukts mit der allgemeinen Formel IV der Bildung eines offenkettigen Säureamids nachgelagert ist.

Die Reste R^{x}, R^{y}, R^{a} und R^{b} sind zu den entsprechenden Resten im ursprünglich eingesetzten Lacton der allgemeinen Formel II und Diamin der allgemeinen Formel III identisch.

Um besonders hohe Ausbeuten an bicyclischem Amidin der allgemeinen Formel I zu erhalten, ist es daher sinnvoll, die Reaktion bis zum im wesentlichen vollständigen Umsatz des Zwischenprodukts der allgemeinen Formel IV zum bicyclischen Amidin der allgemeinen Formel I fortzuführen. Das erfindungsgemäße Verfahren zeichnet sich daher in einer bevorzugten Ausführungsform dadurch aus, daß die Reaktion so lange fortgeführt wird, bis ein im wesentlichen vollständiger Umsatz des Zwischenprodukts der allgemeinen Formel IV zum gewünschten, bicyclischen Amidin der allgemeinen Formel I stattgefunden hat.

Es sind im Rahmen des erfindungsgemäßen Verfahrens jedoch auch alle Zwischenstufen in der Reaktionsführung denkbar. Das heißt, daß durch Variation der Reaktionsführung das im Reaktionsgemisch vorliegende Verhältnis von Zwischenprodukt der allgemeinen Formel IV und bicyclischem Amidin der allgemeinen Formel I gesteuert werden kann.

Das erfindungsgemäße Verfahren läßt sich durch Anpassung der Reaktionsdauer und der Reaktionstemperatur so lenken, daß das als Zwischenprodukt anfallende Diazacycloalken der allgemeinen Formel IV in hohen Ausbeuten isolierbar ist.

Gegenstand der Erfindung ist demnach auch ein Verfahren zur Herstellung eines Diazacycloalkens der allgemeinen Formel IV, bei dem ein Reaktionsgemisch enthaltend ein Lacton der allgemeinen Formel II und ein Diamin der allgemeinen Formel III unter Wasserabspaltung, beispielsweise bei einer Reaktionstemperatur von 80°C bis 35°C, zur Reaktion gebracht wird, wobei während der Reaktionsdauer durch die Wasserabspaltung entstandenes Wasser aus dem Reaktionsgemisch entfernt wird und die Reaktion zu einem Zeitpunkt abgebrochen wird, zu dem das Diazacycloalken der allgemeinen Formel IV im Reaktionsgemisch vorliegt.

Der Zeitpunkt zu dem die Reaktion abgebrochen wird bestimmt sich beispielsweise nach der gewünschten Ausbeute an Diazacycloalken der allgemeinen Formel IV. Im vorliegenden Fall nimmt die Menge an Diazacycloalken im Reaktionsgemisch zu Beginn der Reaktion zunächst zu, während sie mit fortschreitender Reaktionsdauer durch Umsetzung des Diazacycloalkens zum bicyclischen Amidin wieder abnimmt. Ein Konzentrationsprofil einer solchen Reaktionskette läßt sich beispielsweise durch analytische Verfolgung des Reaktionsverlaufs in bestimmten zeitlichen Abständen anschaulich darstellen.

Um das erfmdungsgemäße Diazacycloalken zu erhalten, ist es daher beispielsweise vorteilhaft, die Reaktion zunächst in festgelegten Zeitabschnitten mittels einer geeigneten Analysemethode zu verfolgen, um festzustellen bei welcher Temperatur und nach welcher Reaktionszeit ein Maximum an Diazacycloalken im Reaktionsgemisch vorliegt. Eine geeignete Analysemethode stellt beispielsweise die Gaschromatographie dar. Wird das erfindungsgemäße Herstellungsverfahren auf ein Reaktionsgemisch angewandt, in dem entweder das Lacton oder das Diamin, oder gegebenenfalls beides, bezüglich seiner Reaktivität im erfindungsgemäßen Herstellungsverfahren noch nicht eingeschätzt werden kann, so empfiehlt es sich, die Umsetzung zunächst mit einer niedrigen Temperatur, beispielsweise in einem Temperaturbereich von etwa 130 - etwa 180°C, vorzugsweise in einem Temperaturbereich von etwa 150 - etwa 170°C, zu beginnen, und anschließend in bestimmten Zeitabständen, beispielsweise alle zwei Stunden, alle vier Stunden oder alle acht Stunden, den Reaktionsverlauf analytisch, beispielsweise gaschromatographisch, zu überprüfen. Bei einer zu geringen Reaktionsgeschwindigkeit, d.h. bei zu langsamer Bildung des Diazacycloalkens der allgemeinen Formel IV, kann anschließend beispielsweise die Reaktionstemperatur erhöht werden, wobei Temperaturschritte von beispielsweise 5°C oder 10°C vorteilhaft sind. Bei einer zu hohen Reaktionsgeschwindigkeit, d.h. bei einer zu schnellen Weiterreaktion des gebildeten Diazacycloalkens zum bicyclischen Amidin, kann die Reaktionstemperatur gegebenenfalls so weit verringert werden, daß die Reaktionsgeschwindigkeit der Diazacycloalkenbildung viel größer ist, als die Reaktionsgeschwindigkeit der Bildung des bicyclischen Amidins.

Auf diese Weise läßt sich bei der Umsetzung von Lactonen und Diaminen gemäß dem erfindungsgemäßen Verfahren, wobei wenigstens eine der beiden Komponenten, die in ihrer Reaktivität nicht eingeschätzt werden kann, auf einfache Weise ein Reaktionsprofil erhalten, nach dem sich anschließend erneute Umsetzungen durchführen lassen.

Zum Erhalt des erfindungsgemäßen Diazacycloalkens der allgemeinen Formel IV kann die Reaktion grundsätzlich zu einem beliebigen Zeitpunkt abgebrochen werden, zu dem das Diazacycloalken im Reaktionsgemisch vorliegt. In der Regel wird dies jedoch zu einem Zeitpunkt geschehen, an dem die im Reaktionsgemisch vorliegende Menge an Diazacycloalken möglichst groß ist, d.h. im Maximum der während des Reaktionsverlaufs entstehenden Menge an Diazacycloalken oder zumindest in der Nähe dieses Maximums. In den Fällen, in denen ein Produktgemisch gewünscht ist, das neben dem Diazacycloalken noch weitere, im Verlauf der Reaktion entstehende Produkte enthält, beispielsweise ein offenkettiges Amid oder das bicyclische Amidin der allgemeinen Formel I, kann die Reaktion zu einem Zeitpunkt abgebrochen werden, zu dem die gewünschten Produkte im gewünschten Verhältnis im Reaktionsgemisch vorliegen. Das Verhältnis der Produkte im Reaktionsgemisch läßt sich durch geeignete analytische Methoden, beispielsweise durch Gaschromatographie, bestimmen.

Für die Herstellung des Diazacycloalkens der allgemeinen Formel IV gelten im wesentlichen die gleichen Bedingungen wie für die Herstellung des bicyclischen Amidins der allgemeinen Formel I, lediglich in Bezug auf Reaktionstemperatur und Reaktionsdauer muß in der Regel im Vergleich zum Herstellungsverfahren für das bicyclische Amidin variiert werden. In Abhängigkeit vom eingesetzten Lacton der allgemeinen Formel II und vom eingesetzten Diamin der allgemeinen Formel III kann die Temperatur variieren, bei der sich überwiegend das Diazacycloalken bildet, eine weitere Reaktion zum bicyclischen Amidin jedoch noch nicht oder nur in untergeordnetem Maße stattfindet. Als grundsätzlicher Anhaltspunkt gilt hierbei, daß mit zunehmender Stabilität des sich bildenden Diazacycloalkens die zur Herstellung des Diazacycloalkens der allgemeinen Formel IV benötigte Reaktionstemperatur sinkt, während mit zunehmender sterischer Hinderung bei der Bildung des Diazacycloalkens eine höhere Temperatur aufgewandt werden muß. In der Regel begünstigen höhere Temperaturen die weitere Reaktion zum bicyclischen Amidin, während niedrigere Temperaturen zwar eine verlängerte Reaktionsdauer bedeuten, jedoch mit größerer Selektivität zum Diazacycloalken führen und eine weitere Reaktion zum bicyclischen Amidin weitestgehend vermieden wird.

Setzt man beispielsweise unsubstituierte Lactone mit unsubstituierten Diaminen um, so wählt man als Reaktionstemperatur vorteilhafterweise eine Temperatur im Bereich zwischen etwa 61°C und etwa 200°C, beispielsweise 80°C, 100°C, 120°C, 130°C, 150°C, 160°C, 170°C, 180°C oder 190°C, wobei Reaktionszeiten zwischen etwa 8 Stunden und etwa 24 Stunden vorteilhaft sind.

In einer weiteren Ausführungsform der Erfindung, sowohl bei der Herstellung des bicyclischen Amidins als auch bei der Herstellung des Diazacycloalkens, wird die Reaktionstemperatur während der Reaktionsdauer gesteigert. Dies kann insbesondere dann sinnvoll sein, wenn unter Inkaufnahme längerer Reaktionszeiten besonders schonend und in hohen Ausbeuten zum Diazacycloalken führend gearbeitet werden soll. So kann beispielsweise die gesamte Reaktionsdauer in zwei bis etwa 6 Phasen, vorzugsweise in etwa 3 bis 4 Phasen eingeteilt werden, in denen die Reaktionstemperatur um jeweils etwa 2°C bis etwa 30°C, insbesondere etwa 5°C bis etwa 15°C erhöht wird.

Das erfindungsgemäße Verfahren zur Herstellung der bicyclischen Amidine der allgemeinen Formel I und das erfindungsgemäße Verfahren zur Herstellung der Diazacycloalkene der allgemeinen Formel IV wird in der Regel bei einem Druck von etwa 0,1 bis etwa 1,5 bar durchgeführt. Vorzugsweise erfolgt die Durchführung jedoch drucklos, d. h., bei Umgebungsdruck oder unter einem geringen Vakuum.

Ein weiterer Gegenstand der Erfindung ist auch ein Diazacycloalken der allgemeinen Formel IV 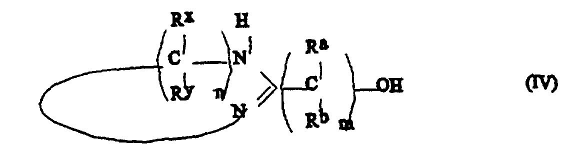 worin R^{a}, R^{b}, R^{x}, R^{y}, n und m die oben genannten Bedeutungen haben.
Ebenfalls Gegenstand der Erfindung ist die Verwendung von Diazacycloalkenen der allgemeinen Formel IV zur Herstellung von bicyclischen Amidinen der allgemeinen Formel I.

Die Erfindung wird nachfolgend durch Beispiele erläutert.

### Beispiele

### Beispiel 1: DBN

In einem 1 l-Kolben, versehen mit Magnetrührer, Tropftrichter, Heizbad und einer 100 cm-Kolonne (gefüllt mit V2A-Ringen) wurden 489 g (6.6 mol) 1,3-Diaminopropan (DAP) vorgelegt und zum Sieden gebracht (135°C). Bei einer Innentemperatur von max. 190°C wurden 353.6 g (4.11 mol) γ-Butyrolacton zugetropft (Molverhältnis 1 : 1.6). Anschließend wurde unter Abdestillieren frisches Diaminopropan zugeführt, um das Molverhältnis in etwa konstant zu halten (siehe Tabelle 1). Auf diese Weise wurde mit dem DAP als Schleppmittel das anfallende Reaktionswasser abdestilliert.

**Tabelle 1**

| Kopftemp. [°C] | Sumpftemp. [°C] | DAP neu [g] | Destillat [g] | H₂O i. Dest. ^{(*)} | Amid ^{a)} [Fl.-%] | Dch ^{(**)}, ^{a)} [Fl.-%] | DBN ^{a)} [Fl.-%] |
|---|---|---|---|---|---|---|---|
| 125 | 190 | 216 | 375 | 56 | 80 | 18 | 2 |
| 130 | 200 | 170 | 192 | 68 | 30 | 60 | 10 |
| 135 | 210 | 175 | 229 | 70 | 10 | 20 | 70 |

| | | | | | | | |
|---|---|---|---|---|---|---|---|
| (*) [mol-% d. Th.] | | | | | | | |
| (**) Diazacyclohexen | | | | | | | |
| a) GC-Analyse des Sumpfes | | | | | | | |

Die Synthese wurde abgebrochen und der Rohaustrag über eine 20 cm Vigreux-Kolonne destilliert. Man erhielt 228 g DBN mit einer Reinheit von > 97 %.

Die Identität des Zwischenproduktes 2-(3-Hydroxypropyl)-1,3-diazacyclohex-1-en wurde durch NMR-Analyse nach Umkristallisieren bewiesen:
¹H-NMR: m (4H) 1.7-1.9 ppm, t (2H) 2.21 ppm, m (4H) 3.22 ppm, t (2H) 3.60 ppm, s (1H, br.) 6.25 ppm, s (1H) 7.63 ppm
¹³C-NMR: 20.4 ppm, 30.1 ppm, 33.1 ppm, 41.1 ppm, 61. 2 ppm, 159.8 ppm
Schmelzpunkt: 96°C, tertiäre Aminzahl: 368 (93 % d. Th.), OH-Zahl: 387 (98 % d. TH.)

### Beispiel 2: DBN

Analog zu Beispiel 1 wurden 25,4 mol Butyrolacton mit 35 mol 1,3-DAP (Molverhältnis 1.38) umgesetzt. Nach 32 h Reaktionszeit betrug die Sumpftemperatur 220°C und 73 % des theoretisch möglichen Wassers waren abdestilliert. Nach Destillation wurden 12 mol DBN mit einer Reinheit von mehr als 99.1 % erhalten. Die Gesamtausbeute an DBN unter Einschluß der in den Destillationsrückständen noch enthaltenen Menge betrug 83.3 %.

### Beispiel 3: 2-(4-Hydroxybutyl)-1,3-diazacyclohex-1-en

Analog Beispiel 1 wurde Valerolacton mit 1,3-DAP umgesetzt. Bei einer Sumpftemperatur von 170°C wurden nach 8 Stunden 48 % des offenkettigen Amids und 49 % 2-(4-Hydroxybutyl)-1,3-diazacyclohex-1-en erhalten. Nach weiteren 8 Stunden bei 180°C und 8 Stunden bei 190°C Sumpftemperatur verschob sich das Verhältnis weiter zu 5 % Amid und 92 % 2-(4-Hydroxybutyl)-1,3-diazacyclohex-1-en. Das Folgeprodukt Diazabicyclodecen wurde erst in einer Menge von 1 bzw. 3 % erhalten.

Die Identität des 2-(4-Hydroxybutyl)-1,3-diazacyclohex-1-ens wurde durch NMR-Analyse nach Umkristallisieren bewiesen:
¹H-NMR: m (2H) 1.55 ppm, m (2H) 1.63 ppm, m (2H) 1.75 ppm, t (2H) 2.12 ppm, m (4H) 3.30 ppm, t (2H) 3.59 ppm, s (1H, br.) 5.65 ppm, s (1H) 7.47 ppm
¹³C-NMR: 20.4 ppm, 23.6 ppm, 31.8 ppm, 35.3 ppm, 41.1 ppm, 61.0 ppm, 159.7 ppm
Tertiäre Aminzahl: 342 (95 % d. Th.), OH-Zahl: 351 (98 % d. Th.)

### Beispiel 4: 2-(4-Hydroxypentyl)-1,3-diazacyclohex-1-en

Analog Beispiel 1 wurde Caprolacton mit 1,3-DAP umgesetzt. Bei einer Sumpftemperatur von 170°C wurden nach 8 Stunden 89 % des offenkettigen Amids und 10 % 2-(4-Hydroxypentyl)-1,3-diazacyclohex-1-en erhalten. Nach weiteren 8 Stunden bei 180°C und 8 Stunden bei 190°C Sumpftemperatur verschob sich das Verhältnis weiter zu 8 % Amid und 89 % 2- (4-Hydroxypentyl)-1,3-diazacyclohex-1-en. Das Folgeprodukt Diazabicycloundecen (DBU) wurde erst in einer Menge von 1 bzw. 3 % erhalten.

Die Identität des 2-(4-Hydroxypentyl)-1,3-diazacyclohex-1-ens wurde durch GC/MS sowie NMR-Analyse nach Umkristallisieren (das Produkt enthielt noch geringere Mengen Verunreinigungen) bewiesen:
¹H-NMR: m (2H) 1.35 ppm, m (4H) 1.55 ppm, m (2H) 1.75 ppm, t (2H) 2.08 ppm, m (4H) 3.26 ppm, t (2H) 3.58 ppm, s (1H, br.) 4.82 ppm, s (1H) 7.41 ppm
¹³C-NMR: 20.5 ppm, 25.5 ppm, 27.1 ppm, 32.2 ppm, 36.2 ppm, 41.3 ppm, 61.8 ppm, 156.6 ppm

## Patentansprüche

1. Verfahren zur Herstellung eines bicyclischen Amidins der allgemeinen Formel I 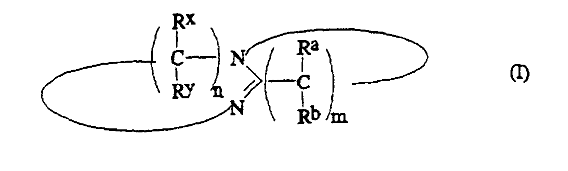 worin R^{x}, R^{y}, R^{a} und R^{b} jeweils unabhängig voneinander für Wasserstoff, C₁₋₁₀-Alkyl, C₂₋₁₀-Alkenyl, C₂₋₁₀-Alkinyl oder C₆₋₁₂-Aryl, wobei die Reste ihrerseits noch mit Hydroxy-, Amino-, C₁₋₄-Alkylamino- oder Mercaptogruppen substituiert sein können, stehen und n und m unabhängig voneinander für Zahlen von 2 bis 12 stehen, wobei a, b, x und y jeweils die Reste R am jeweiligen Kohlenstoffatom 1 bis m bzw. 1 bis n indizieren,
bei dem ein Reaktionsgemisch, enthaltend ein Lacton der allgemeinen Formel II 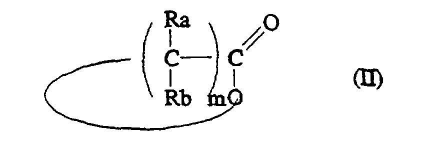 worin R^{a}, R^{b} und m jeweils die oben genannte Bedeutung haben,
und ein Diamin der allgemeinen Formel III 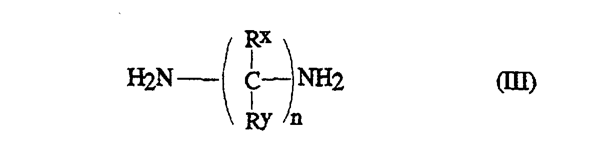 worin R^{x}, R^{y} und n jeweils die oben genannte Bedeutung haben,
unter Wasserabspaltung zur Reaktion gebracht wird, **dadurch gekennzeichnet, daß** durch die Wasserabspaltung entstandenes Wasser während der Reaktion aus dem Reaktionsgemisch entfernt wird.

2. Verfahren nach Anspruch 1, **dadurch gekennzeichnet, daß** als Zwischenprodukt ein Diazacycloalken der allgemeinen Formel IV 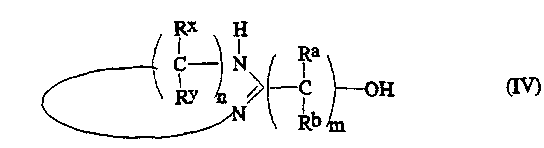 worin R^{x}, R^{y}, R^{a} und R^{b} die in Anspruch 1 genannte Bedeutung haben,
entsteht, und die Reaktion so lange fortgeführt wird, bis ein im wesentlichen vollständiger Umsatz des Zwischenprodukts zum bicyclischen Amidin der allgemeinen Formel I stattgefunden hat.

3. Verfahren nach Anspruch 1 oder 2, **dadurch gekennzeichnet, daß** die Entfernung des durch die Wasserabspaltung entstandenen Wassers über einen Zeitraum erfolgt, der mindestens der Hälfte der gesamten Reaktionsdauer entspricht.

4. Verfahren nach Anspruch 1 bis 3, **dadurch gekennzeichnet, daß** das durch die Wasserabspaltung entstandene Wasser mit Hilfe eines Schleppmittels entfernt wird.

5. Verfahren nach einem der Ansprüche 1 bis 4, **dadurch gekennzeichnet, daß** dem Reaktionsgemisch während der Reaktionsdauer mindestens ein Diamin der allgemeinen Formel III zugeführt wird.

6. Verfahren nach einem der Ansprüche 1 bis 5, **dadurch gekennzeichnet, daß** einer oder mehrere der folgenden Verfahrensparameter eingehalten werden:
a) die Reaktionstemperatur beträgt 170°C bis 220°C,
b) die Reaktionsdauer beträgt 5 Stunden bis 100 Stunden,
c) die Entfernung des Wassers aus dem Reaktionsgemisch erfolgt kontinuierlich während der gesamten Reaktionsdauer,
d) die Reaktion wird bei einem Druck zwischen 0,1 und 1,5 bar, vorzugsweise bei Umgebungsdruck, durchgeführt.

7. Diazacycloalkene der allgemeinen Formel IV 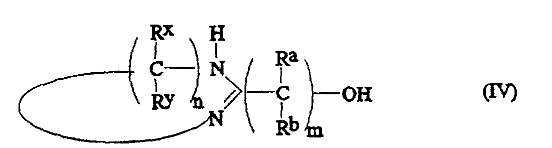 worin R^{x}, R^{y}, R^{a} und R^{b} die in Anspruch 1 angegebene Bedeutung haben.

8. Verfahren zur Herstellung eines Diazacycloalkens der allgemeinen Formel IV, bei dem ein Reaktionsgemisch, enthaltend ein Lacton der allgemeinen Formel II und ein Diamin der allgemeinen Formel III unter Wasserabspaltung zur Reaktion gebracht wird, **dadurch gekennzeichnet, daß** durch die Wasserabspaltung entstandenes Wasser während der Reaktion aus dem Reaktionsgemisch entfernt wird und die Reaktion zu einem Zeitpunkt abgebrochen wird, zu dem das Diazacycloalken der allgemeinen Formel IV im Reaktionsgemisch vorliegt.

9. Verfahren nach Anspruch 8, **dadurch gekennzeichnet, daß** die Reaktionstemperatur 80°C bis 350°C, vorzugsweise 120°C bis 220°C beträgt.

10. Verwendung eines Diazacycloalkens der allgemeinen Formel IV zur Herstellung eines bicyclischen Amidins der allgemeinen Formel I.

## Claims

1. A process for preparing a bicyclic amidine of the formula I 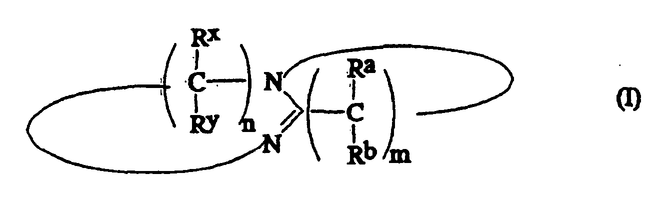 where R^{x}, R^{y}, R^{a} and R^{b} are each, independently of one another, hydrogen, C₁-C₁₀-alkyl, C₂-C₁₀-alkenyl, C₂-C₁₀-alkynyl or C₆-C₁₂-aryl, where the radicals can in turn be further substituted by hydroxy, amino, C₁-C₄-alkylamino or mercapto groups, and n and m are, independently of one another, numbers from 2 to 12, where a, b, x and y each index the radicals R on the respective carbon atom 1 to m or 1 to n,
by reacting a reaction mixture comprising a lactone of the formula II 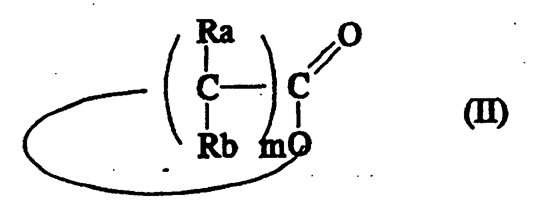 where R^{a}, R^{b} and m are each as defined above,
and a diamine of the formula III 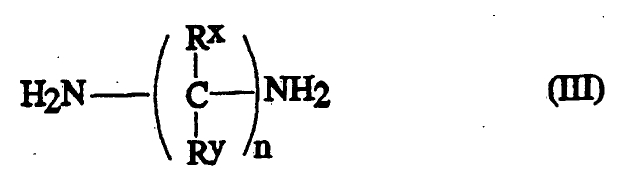 where R^{x}, R^{y} and n are each as defined above,
with elimination of water, wherein water formed by elimination is removed from the reaction mixture during the reaction.

2. A process as claimed in claim 1, wherein a diazacycloalkene of the formula IV 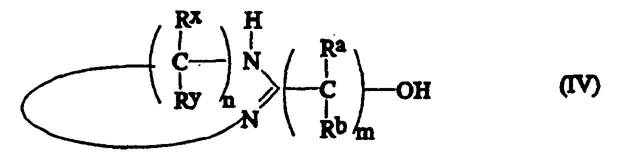 where R^{x}, R^{y}, R^{a} and R^{b} are as defined in claim 1,
is formed as an intermediate and the reaction is continued until essentially complete conversion of the intermediate into the bicyclic amidine of the formula I has taken place.

3. A process as claimed in claim 1 or 2, wherein the water formed by elimination is removed over a period of time which corresponds to at least half the total reaction time.

4. A process as claimed in any of claims 1 to 3, wherein the water formed by elimination is removed with the aid of an entrainer.

5. A process as claimed in any of claims 1 to 4, wherein at least one diamine of the formula III is fed into the reaction mixture during the reaction.

6. A process as claimed in any of claims 1 to 5, wherein one or more of the following process parameters is/are adhered to:
a) the reaction temperature is from 170°C to 220°C,
b) the reaction time is from 5 hours to 100 hours,
c) the water is removed continuously from the reaction mixture during the entire reaction time,
d) the reaction is carried out at a pressure of from 0.1 to 1.5 bar, preferably at ambient pressure.

7. A diazacycloalkene of the formula IV 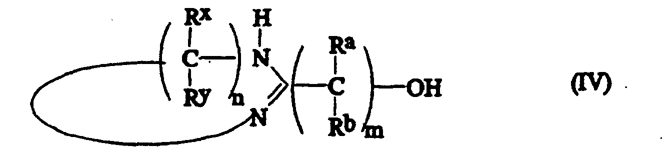 where R^{x}, R^{y}, R^{a} and R^{b} are as defined in claim 1.

8. A process for preparing a diazacycloalkene of the formula IV by reacting a reaction mixture comprising a lactone of the formula II and a diamine of the formula III with elimination of water, wherein water formed by elimination is removed from the reaction mixture during the reaction and the reaction is stopped at a point in time at which the diazacycloalkene of the formula IV is present in the reaction mixture.

9. A process as claimed in claim 8, wherein the reaction temperature is from 80°C to 350°C, preferably from 120°C to 220°C.

10. The use of a diazacycloalkene of the formula IV for preparing a bicyclic amidine of the formula I.

## Revendications

1. Procédé pour la préparation d'une amidine bicyclique de formule générale I 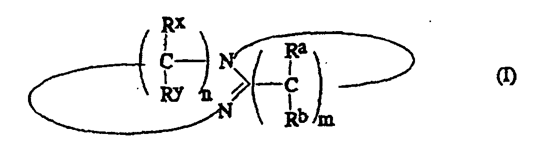 dans laquelle R^{x}, R^{y}, R^{a} et R^{b} représentent chacun, indépendamment l'un de l'autre, un atome d'hydrogène, un groupe alkyle en C₁ à C₁₀, alcényle en C₂ à C₁₀, alcynyle en C₂ à C₁₀ ou aryle en C₆ à C₁₂, ces restes pouvant également être substitués eux-mêmes par des groupes hydroxyle, amine, (alkyle en C₁ à C₄)amine ou mercapto, et n et m représentent, indépendamment l'un de l'autre, un nombre compris dans la plage de 2 et 12, où a, b, x et y indicent respectivement les restes R situés sur les 1 à m, respectivement 1 à n, atomes de carbone correspondant,
en faisant réagir, avec élimination d'eau, un mélange réactionnel contenant une lactone de formule générale II 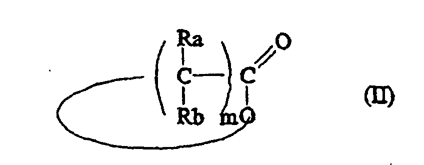 dans laquelle R^{a}, R^{b} et m prennent chacun la signification susmentionnée, et une diamine de formule générale III 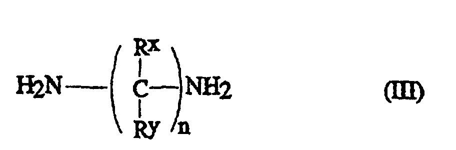 dans laquelle R^{x}, R^{y} et n prennent chacun la signification susmentionnée,
**caractérisé en ce que** l'eau formée par l'élimination d'eau est séparée du mélange réactionnel pendant la réaction.

2. Procédé selon la revendication 1, **caractérisé en ce que** l'on forme, en tant que produit intermédiaire, un diazacycloalcène de formule générale IV 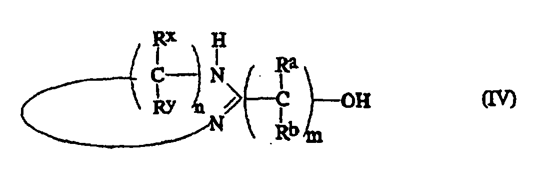 dans laquelle R^{x}, R^{y}, R^{a} et R^{b} prennent la signification mentionnée dans la revendication 1,
et **en ce que** l'on poursuit la réaction jusqu'à ce que la réaction du produit intermédiaire en amidine bicyclique de formule générale I soit, pour l'essentiel, complètement achevée.

3. Procédé selon la revendication 1 ou 2, **caractérisé en ce que** l'on réalise la séparation de l'eau formée par l'élimination d'eau, pendant une durée qui correspond au moins à la moitié de la durée totale de la réaction.

4. Procédé selon les revendications 1 à 3, **caractérisé en ce que** l'on réalise la séparation de l'eau formée par l'élimination d'eau, au moyen d'un agent d'entraînement.

5. Procédé selon l'une quelconque des revendications 1 à 4, **caractérisé en ce que** l'on ajoute au mélange réactionnel, pendant la durée de la réaction, au moins une diamine de formule générale III.

6. Procédé selon l'une quelconque des revendications 1 à 5, **caractérisé en ce que** l'on respecte un ou plusieurs paramètres du procédé suivants :
a) la température de réaction est comprise entre 170°C et 220°C,
b) la durée de la réaction est comprise entre 5 heures et 100 heures,
c) on réalise la séparation de l'eau du mélange réactionnel en continu pendant toute la durée de la réaction,
d) on procède à la réaction à une pression comprise entre 0,1 bar et 1,5 bar, de préférence à pression ambiante.

7. Diazacycloalcènes de formule générale IV 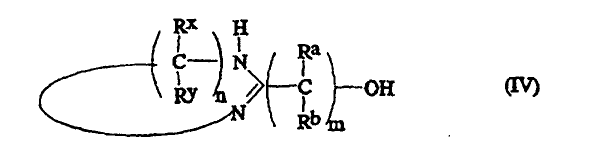 dans laquelle R^{x}, R^{y}, R^{a} et R^{b} prennent la signification mentionnée dans la revendication 1.

8. Procédé pour la préparation des diazacycloalcènes de formule générale IV, dans lequel on fait réagir un mélange réactionnel contenant une lactone de formule générale II et une diamine de formule générale III, avec élimination d'eau, **caractérisé en ce que** l'on sépare du mélange réactionnel, pendant la réaction, l'eau formée par l'élimination d'eau, et **en ce que** l'on arrête la réaction à un moment où le diazacycloalcène de formule générale IV est présent dans le mélange réactionnel.

9. Procédé selon la revendication 8,**caractérisé en ce que** la température de la réaction est comprise entre 80°C et 350°C, de préférence entre 120°C et 220°C.

10. Mise en oeuvre d'un diazacycloalcène de formule générale IV pour la préparation d'une amidine bicyclique de formule générale I.
